# EUROPEAN PATENT SPECIFICATION

(11) **EP 1 380 561 B1**
(45) Date of publication and mention of the grant of the patent: **19.03.2008**
(21) Application number: 02015441.5
(22) Date of filing: 11.07.2002
(51) Int. Cl.: C07C 45/85, C07C 49/08, C07C 45/53

(54) **Continuous process for recovering acetone from a waste stream resulting from acetone purification**
Kontinuierliches Verfahren zur Rückgewinnung von Aceton aus einem Abfallstrom der Acetonreinigung
Procédé continu de recyclage d'acétone à partir d'un flux résiduaire issu de la purification de l'acétone

(43) Date of publication of application: 14.01.2004
(73) Proprietor: INEOS Phenol GmbH & Co. KG, 45966 Gladbeck (DE)
(72) Inventor: Weber, Markus, Dr., 45721 Haltern (DE); Schnurr, Otto, Dr., 2950 Putte-Kapellen (BE)
(74) Representative: Polypatent

(56) References cited:
- EP-A- 1 188 737
- GB-A- 1 108 327

## Description

The present invention relates to a continuous process for recovering acetone from a waste stream resulting from an acetone purification stage. In particular, the present invention relates to a process for the manufacture of phenol and acetone from cumene according to the Hock process wherein in one step acetone is recovered in a continuous process from a waste stream of an acetone purification stage by cleavage of mesityl oxide contained therein.

It is known from the state of the art that mesityl oxide can be reversed into acetone from which it is formed by treatment under acidic or basic conditions. For example, US Patent No. 2,351,352 discloses a process for producing isophorone by condensing acetone whereas mesityl oxide which is formed inter alia as a by-product during this reaction is reversed into acetone by commingling the crude product mixture with aqueous sodium hydroxide solution, followed by distilling the thus obtained acetone in one step together with unreacted acetone from the main reaction. This prevents forming further mesityl oxide in case of a preliminary removal of unreacted acetone.

The manufacture of phenol and acetone which is of great economic importance typically proceeds starting from cumene according to the Hock reaction by acid catalyzed cleavage of cumene hydroperoxide. The by-products formed during this reaction, like for example, cumene, benzene and one or more ketones, have to be separated from the main products phenol and acetone, which generally proceeds via extraction and/or distillation.

Often an acetone stream obtained from a preceding distillation to separate it from phenol, is treated with alkaline reagents to further separate it from undesired by-products, especially aldehydes. Because of these acidic and alkaline reaction conditions, respectively, the acetone partly reacts with itself to form diacetone alcohol in a first step which may either dehydrate to form mesityl oxide or decompose again to redevelop acetone.

Since acetone represents one of the desired products a procedure in which the crude product of the Hock process is directly commingled with an alkaline solution to reverse mesityl oxide contained therein to acetone analogous to US Patent 2,351,352 as mentioned above, is not suitable in this case because under these conditions additional by-products would be formed which leads to a reduction in yield. Moreover, any further acetone purification steps using an alkaline solution also will result in the production of mesityl oxide which accordingly would not be recovered and therefore as well lead to an additional loss of yield.

Accordingly, EP-A-1 188 737 discloses a process for the work-up of substance streams containing mesityl oxide which are especially obtained from acetone purification during the Hock process.

In this process the mesityl oxide is concentrated and reversed to acetone using an acidic or basic aqueous medium or an acidic catalyst in the presence of water.

The disadvantages of this recovery process for mesityl oxide can be seen in the cleavage taking place in a separate reaction apparatus which increases the costs for new plants to be constructed, as well as the costs for the maintenance of such a plant. Additionally, the only process exemplified therein represents a discontinuous one which is susceptible for disorders.

Abstract of JP-A 54 070 210 also deals with acetone recovery from mesityl oxide in the cumene process of phenol manufacture whereas in this process the alkali required for the conversion is a waste liquor from the acetone purification in the cumene processes. Since the cleavage of mesityl oxide described therein proceeds in an autoclave this process as well requires a separate reaction apparatus.

Accordingly the problem underlying the present invention presents as providing a process for recovering acetone from a waste stream from an acetone purification stage which requires less apparative expenses and preferably reduces maintenance costs as well.

This problem is inventively solved by providing a continuous process for recovering acetone from a waste stream from an acetone purification stage said waste stream comprising mesityl oxide, and acetone by
i) separating the waste stream in a separating device at least in one stream comprising mesityl oxide, and in a further stream comprising acetone,
ii) concentrating mesityl oxide in the mesityl oxide containing stream and
iii) recycling the concentrated mesityl oxide stream to the separating device and bringing it into contact with a basic or acidic medium or with an acidic catalyst in the presence of water whereby mesityl oxide is at least partially hydrolyzed to acetone.

A further subject-matter of the present invention is a process to manufacture phenol and acetone from cumene according to the Hock process by:
A) oxidizing cumene to form cumene hydroperoxide,
B) subjecting cumene hydroperoxide to acid catalyzed cleavage obtaining a cleavage phase comprising phenol and acetone,
C) separating phenol and acetone,
D) purifying the crude acetone from step C) resulting in a pure acetone product stream and a waste stream, and
E) recovering acetone from the waste stream according to the above described process.

By developing a continuous process in which the cleavage of the mesityl oxide takes place in the same separating device which is used to separate the mesityl oxide containing, preferably organic, stream from the waste stream, it is possible to reduce the number of reaction columns. This does not only reduce costs when creating a new plant but it also reduces the costs for maintaining the existing plant and therefore being very attractive for industrial purposes. Besides, such a continuous process reduces the man power required for a smooth processing. Especially the reconduction of the separated purified products into the main process is omitted in the inventive process. In a preferred embodiment a basic aqueous medium which is already present in the waste stream is used to ease the cleavage of mesityl oxide, which leads to further cost reduction and decreases waste disposal.

The mesityl oxide containing waste stream may be obtained by any kind of acetone purification. Preferably the waste stream results from the acetone purification of crude acetone which is most preferably obtained from the Hock process. Beyond, this waste stream may as well represent a mixture of several side streams obtained during varying purification procedures. The crude acetone may be purified by separating it according to common procedures, for example, in a reaction-distillation column wherein the pure acetone is removed, preferably as a top stream leaving behind the waste stream preferably as a bottom stream.

The inventive process makes use of the reverse of the known reaction in which two molecules of acetone react under basic or acidic conditions in an Aldol reaction to diacetone alcohol which further dehydrates to mesityl oxide. Accordingly, to recover acetone from mesityl oxide-containing waste streams this reaction is reversed which requires to bring the mesityl oxide-containing waste stream into contact with an aqueous medium which may either be basic or acidic or with an acidic catalyst in the presence of water to catalyze the hydration reaction. Such a catalyst is preferably an immobilized heterogeneous catalyst, for example, a zeolite or an acid ion-exchange resin.

As acidic medium preferably mineral acids or organic acids like hydrochloric acid, sulfuric acid or acidic acid in various concentrations may be used. As basic mediums alkali lyes, like soda lye, or alkaline earth metal lyes as well as organic water containing phases of compounds with basic properties, like amines or phenolates, may be used. Soda lye represents the most preferred basic aqueous medium.

The basic or acidic aqueous medium or the acidic catalyst containing water may be either separately fed to the separating device or be already present in the waste stream. If the aqueous medium is separately fed to the separating device preferably a basic solution containing 1-10 wt% sodium hydroxide, more preferably containing 4 wt% sodium hydroxide, is used. It should be further provided that the amount of acidic or basic aqueous medium or acidic catalyst containing water fed to the separating device results in a reaction mixture of waste stream and aqueous medium containing 5-15 percent by volume aqueous medium, based on the total volume of the reaction mixture.

In the preferred embodiment in which the aqueous medium is already present in the waste stream the concentration of the aqueous medium in the waste stream is preferably ≥ 0,1 percent by volume, more preferably 0,1 to 5 percent by volume, most preferred from 0,1 to 0,2 percent by volume, based on the total volume of the waste stream.

Since at least the recycled mesityl oxide, optionally further components as well, may only be dissolved to a small extent in water, or are not soluble in water at all, to achieve an optimized turn-over of the mesityl oxide cleavage reaction it should be provided that the organic and aqueous phases are intensively agitated which may be obtained with common means, preferably making use of pumps, stirrers or a reboiler.

A great advantage of conducting the process in a manner that preferably a basic aqueous medium is already present in the waste stream, especially a basic aqueous medium resulting from the usage in a preceding acetone purification step, can be seen in reducing the amount of basic aqueous medium, especially the solution of sodium hydroxide, required for the whole process, and in connection with this in reducing the expense for waste disposal.

Concentration of the mesityl oxide-containing stream may be achieved by common methods, for example, by extraction or distillation, preferably by means of distillation. The concentrated mesityl oxide stream preferably has a mesityl oxide content of more than 20 wt%, more preferred from 30-90 wt%, and most preferred from 50-85 wt%.

In an especially preferred embodiment the process proceeds using a waste stream which contains additionally to mesityl oxide and optional acetone one or more organic components having a boiling point higher than acetone which in case that the process represents a part of the work-up stages of the Hock process, for example, may comprise cumene, benzene and/or ketones. In this embodiment a basic medium is chosen as hydrating medium. The recovery of acetone is then achieved by
a) continuously feeding said waste stream to a middle section of a first distillation column;
b) separating the waste stream in the first distillation column in
   b1) optionally a top stream comprising acetone;
   b2) a side stream comprising mesityl oxide, the organic components and optionally residual acetone; and
   b3) a bottom stream comprising the basic aqueous medium;
c) continuously feeding side stream b2) to a middle section of a second distillation column;
d) separating the side stream b2) in the second distillation column in
   d1) a top stream comprising acetone;
   d2) a side stream comprising mesityl oxide; and
   d3) a bottom stream comprising organic components having a boiling point higher than mesityl oxide; and
e) continuously recycling side stream d2) to the first distillation column to an entry point that is below the removal point for side stream b2).

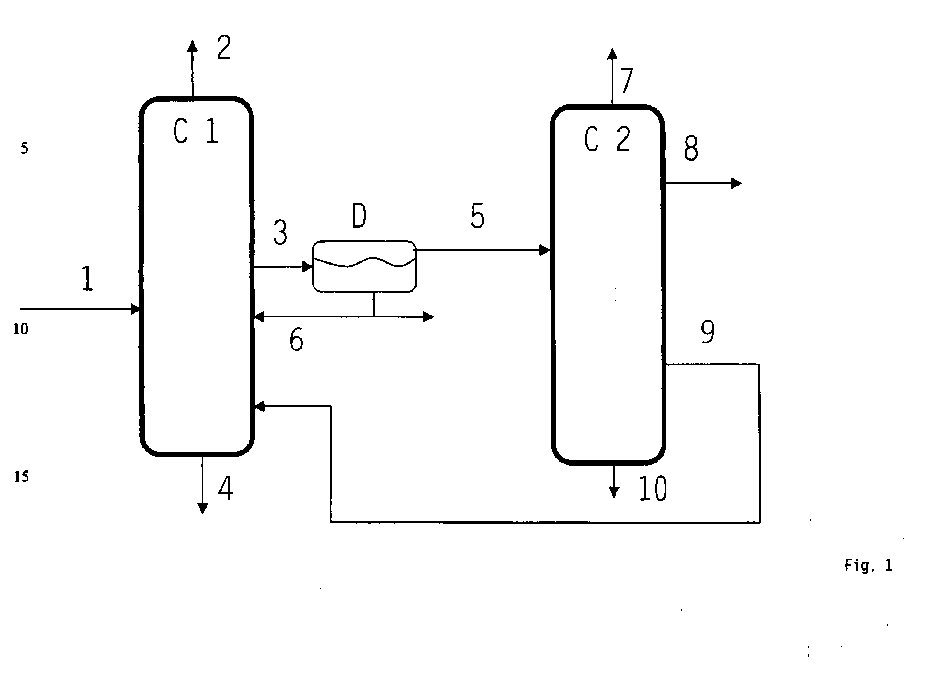

This process is explained in more detail in combination with the reaction parameters according to Figure 1.

Fig. 1 shows a schematic representation of a preferred embodiment of the present invention.

A waste stream containing mesityl oxide and optionally acetone is continuously fed via line 1 into a middle section of a first distillation column C 1 which functions as separating device. The basic aqueous medium preferably containing sodium hydroxide may result from treatment of crude acetone, for example obtained by the Hock process, with aqueous sodium hydroxide solution to remove aldehydes. The separation in the distillation column C 1 may proceed with support of common column internals, like reaction trays, hackings and trickle columns, whereas in the preferred embodiment reaction trays are used.

In said first distillation column C 1 the waste stream is separated, optionally into a top stream comprising acetone and eventually traces of high boiling hydrocarbons which is removed via line 2, into a side stream comprising mesityl oxide, the organic components and optionally residual acetone removed via line 3 and into a bottom stream comprising the basic aqueous medium partly removed via line 4. The residual acetone removed via line 2 may be transferred back to the separating device from which the used waste stream resulted, which preferably represents the distillation column in which crude acetone from the Hock process is separated into pure acetone and the waste stream. Of course it is as well possible to combine several acetone streams occurring anywhere during the work-up procedure of the crude product mixture. The part of the basic aqueous medium contained in the bottom stream which is not required for reversing mesityl oxide to acetone may be transferred to the separating device in which the crude acetone is purified using a basic aqueous medium or be removed, optionally after having been neutralized, as waste water.

According to the preferred embodiment shown in Fig. 1 the side stream removed via line 3 comprising mesityl oxide, the organic components, water and optionally residual acetone is continuously fed to a decanter D, where the aqueous phase and the organic phase are separated. If the distillation column C 1 is run in a way that the side stream removed via line 4 does not contain water or only negligible amounts of water the decanter can be omitted. The aqueous phase is at least partially recycled to the distillation column C 1 via line 6, or can be discarded as waste water stream.

The organic phase from decanter D comprising mesityl oxide, the organic components and optionally residual acetone is continuously fed via line 5 to a middle section of a second distillation column C 2. The column interiors of said second distillation column may be the same or different from the ones used in the first distillation column C 1. In said second distillation column side stream from the first distillation column C 1 is separated into a top stream comprising optionally acetone which is removed via line 7, into a side stream comprising mesityl oxide which is removed via line 9 and into a bottom stream comprising organic components having a boiling point higher than mesityl oxide which is removed via line 10.

Analogous to the acetone top stream obtained in the first distillation column the acetone stream of the second distillation column may be further worked up and combined with the pure acetone obtained from any other acetone purification step, preferably it is transferred to the distillation column serving as separating device in which the crude acetone obtained from the Hock process is separated into pure acetone and the waste stream. The mixture of bottom stream components may be either further separated, worked up or transferred to combustion, the resulting energy of which may be used anywhere in the process, for example to heat the distillation columns. Depending on its composition it might be meaningful to transfer the obtained bottom stream to the work-up stage of the final residue of the main reaction.

The side stream removed via line 9 comprising mesityl oxide is continuously recycled to the first distillation column C 1 to an entry point that is below the removal point of line 3. In a preferred embodiment in which the first distillation column C 1 is equipped with reaction trays the recycled mesityl oxide stream is directed onto one of these reaction trays.

The recycled mesityl oxide is brought into contact with the basic aqueous medium into an area of the first distillation column C 1 in which beside optional residual acetone at least one further organic component can be found which, like mesityl oxide, may only be dissolved to a small extent in water, or which is not soluble in water at all. To achieve an optimized turn-over of the mesityl oxide cleavage reaction it should be provided that the organic and aqueous phases are intensively agitated. This agitation may be obtained by common means, preferably making use of pumps, stirrers or a reboiler.

To ease the reaction the cleavage reaction mixture of mesityl oxide and basic aqueous medium while being agitated should preferably be thermally treated. Accordingly the temperature at the entry point of the recycled mesityl oxide stream should be 30 to 160°C, preferably 70 to 110 °C.

In an especially preferred embodiment the second distillation column C 2 provides a second side stream that is removed via line 8 comprising ketones and optionally further organic compounds with a boiling point lower than mesityl oxide and higher than acetone, like for example benzene, and which is positioned upwards relative to the position of line 9. It is a particular advantage of the process of the present invention that compounds that are detrimental to the acetone quality especially benzene can be easily removed from the process streams without additional separation stages. Said second side stream may be further worked up and separated or transferred to combustion, for example, to use the thus resulting energy for heating the distillation columns.

The bottom stream removed via line 10 from the second distillation column C 2 comprises the high boiling hydrocarbons like cumene. Preferably cumene is recovered from said stream and recycled to the cleavage stage of the Hock process. Alternatively the bottom stream may be transferred to combustion to utilize the energy content of said stream.

The process of the present invention can be easily integrated into the Hock process for producing phenol. Thus, a further subject of the present invention is a process to manufacture phenol and acetone from cumene according to the Hock process by:
A) oxidizing cumene to form cumene hydroperoxide,
B) subjecting cumene hydroperoxide to acid-catalyzed cleavage obtaining a cleavage phase comprising phenol and acetone,
C) separating phenol and acetone,
D) purifying the crude acetone from step C) resulting in a pure acetone product stream and a waste stream, and
E) recovering acetone from a waste stream according to the continuous process described above.

Preferably the crude acetone of step D) is purified using an aqueous sodium hydroxide solution, followed by distillation to obtain a pure acetone product stream and a waste stream whereby the waste stream contains preferably not more than 30% acetone, water, sodium hydroxide, diacetone alcohol, mesityl oxide, cumene, benzene and ketones, and optionally further organic by-products.

In a preferred embodiment said waste stream mixture is transferred to a first distillation column B) wherein most of the acetone is removed in a top stream and transferred back to the purification stage of the crude acetone while the mixture of organic compounds preferably is transferred to the second distillation column resulting in a bottom stream comprising an aqueous sodium hydroxide solution. In the preferred embodiment the bottom stream of the first distillation column contains beside the basic aqueous medium less than 2wt%, preferably less than 1wt% mesityl oxide and is recycled to the work-up process of the crude acetone stream in the Hock process.

The mixture of organic compounds transferred to the second distillation column is separated into a top stream containing acetone, a side stream containing organic substances with a boiling point lower than mesityl oxide and higher than acetone, like ketones and benzene, a further side stream containing mesityl oxide and a bottom stream containing cumene and optionally other compounds with a boiling point higher than mesityl oxide. The cumene containing bottom stream of the second distillation column is preferably recycled to the Hock process, optionally after preceding separation.

The recovered mesityl oxide is transferred back to the first distillation column at an entry point below the removing line of the organic compound mixture, preferably directly onto one of the reaction trays with which the distillation column is equipped. The temperature at the entry point is preferably 80-90°C.

The present invention shall be exemplified by means of a working example:

### Example 1

A waste stream was obtained from the acetone purification stage of the Hock process comprising 2 wt-% low boiling hydrocarbons, 11 wt-% acetone, 68 wt-% water, 10 wt-% mesityl oxide and 9 wt-% high boiling hydrocarbons and continuously fed to the middle section of a first distillation column as shown in Fig. 1. Based on 1000 parts by weight of waste stream the waste stream was continuously separated into 150 parts by weight of a head product stream consisting essentially of acetone and containing only traces of low boiling hydrocarbons and into 713 parts by weight of a bottom stream consisting essentially of water and some high boiling hydrocarbons. A side stream was removed from the first distillation column and fed to an decanter, wherein the aqueous phase and the organic phase was separated. The aqueous phase was recycled to the first column and 279 parts by weight organic phase based on 1000 parts by weight of the waste stream comprising 7 wt-% low boiling hydrocarbons, 36 wt-% mesityl oxide, 25 wt-% acetone and 32 wt-% high boiling hydrocarbons was continuously fed to a middle section of a second distillation column. The organic phase was continuously separated in 65 parts by weight of a head product stream consisting essentially of acetone, in 25 parts by weight of an upper side stream comprising 80 wt-% low boiling hydrocarbons and 20 wt-% acetone, 47 parts by weight of a bottom stream comprising high boiling hydrocarbons i.a. cumene and 142 parts by weight of a lower side stream comprising 70 wt-% mesityl oxide, 25 wt-% high boiling hydrocarbons and 5 wt-% acetone, all parts by weight being based on 1000 parts by weight of the initial waste stream. With upper side stream benzene was quantitatively removed from the waste stream. The lower side stream was continuously recycled to a lower portion of the first distillation column, where the mesityl oxide was converted to acetone as described above. As can be seen from this example valuable components like acetone, mesityl oxide converted to acetone and cumene can be economically recovered from a waste stream using the process of the present invention.

## Claims

1. A continuous process for recovering acetone from a waste stream from an acetone purification stage, said waste stream comprising mesityl oxide and acetone by
i) separating the waste stream in a separating device at least in one stream comprising mesityl oxide and a further stream comprising acetone;
ii) concentrating mesityl oxide in the mesityl oxide containing stream; and
iii) recycling the concentrated mesityl oxide containing stream to the separating device and bringing it into contact with a basic or acidic aqueous medium or with an acidic catalyst in the presence of water whereby mesityl oxide is at least partially hydrolyzed to acetone.

2. The process of claim 1, wherein concentrating of mesityl oxide is achieved by means of distillation.

3. The process of any one of claims 1 and 2, wherein the concentrated mesityl oxide containing stream contains at least 20, preferably 50 to 85 percent by weight of mesityl oxide.

4. The process of any one of the preceding claims, wherein the separation device is a distillation column.

5. The process of any one of the preceding claims, wherein the basic or acidic aqueous medium is separately fed to the separating device.

6. The process of claim 5, wherein the concentrated mesityl oxide containing stream is mixed with water and is contacted in the separation device with an acidic catalyst, preferably an immobilized heterogeneous catalyst.

7. The process of any one of claims 1 to 4, wherein the basic or acidic aqueous medium is already present in the waste stream.

8. The process of claim 7, wherein the concentration of the basic or acidic medium in the waste stream is ≥ 0.1 percent by volume based on the complete volume of the waste stream.

9. The process of any one of claims 7 and 8, wherein the waste stream comprises in addition one or more organic components having a boiling point higher than acetone, the basic or acidic aqueous medium is a basic medium and recovery of acetone is achieved by
a) continuously feeding said waste stream to a middle section of the first distillation column;
b) separating the waste stream in the first distillation column in
b1) optionally a top stream comprising acetone;
b2) a side stream comprising mesityl oxide, the organic components and optionally residual acetone; and
b3) a bottom stream comprising the basic aqueous medium;
c) continuously feeding side stream b2) to a middle section of the second distillation column;
d) separating the side stream b2) in the second distillation column in
d1) a top stream comprising acetone;
d2) a side stream comprising mesityl oxide; and
d3) a bottom stream comprising organic components having a boiling point higher than mesityl oxide; and
e) continuously recycling side stream d2) to the first distillation column to an entry point that is below the removal point for side stream b2).

10. The process of claim 9, wherein side stream b2) is continuously fed to a decanter, where the side stream is separated in an organic phase which is continuously fed to a middle section of the second distillation column and an aqueous phase which is at least partially recycled to the first distillation column.

11. The process of any of claims 9 and 10, wherein the first distillation column comprises reaction trays and the recycled mesityl oxide containing stream is directed onto one or more of the reaction trays.

12. The process of any one of claims 9 to 11, wherein the temperature at the entry point is 30 to 160°C, preferably 70 to 110°C.

13. The process of any one of claims 9 to 12, wherein in the second distillation column a second side stream d4) comprising organic components having a boiling point lower than mesityl oxide is removed at a position upwards relative to the position of removal of side stream d2).

14. The process of claim 13, wherein side stream d4) comprises benzene.

15. The process of any of the preceding claims, wherein the waste stream results from the acetone purification of crude acetone obtained from the Hock process, whereby crude acetone is separated in a distillation column, wherein pure acetone is removed as top stream and the waste stream as bottom stream.

16. The process of claim 15, wherein any recovered acetone streams are combined and recycled to the acetone purification column.

17. A process to manufacture phenol and acetone from cumene according to the Hock process by:
A) oxidizing cumene to form cumene hydroperoxide;
B) subjecting cumene hydroperoxide to acid-catalyzed cleavage obtaining a cleavage phase comprising phenol and acetone;
C) separating phenol and acetone;
D) purifying the crude acetone from step C) resulting in a pure acetone product stream and a waste stream; and
E) recovering acetone from the waste stream according to a process of any one of claims 1 to 16.

18. The process of claim 17, wherein in the recovery step E) a process according to any of claims 9 to 14 is used and wherein
bottom stream d3) comprises cumene and is recycled to the Hock process and
bottom stream b3) comprises the basic aqueous medium and less than 2 percent by weight, preferably less than 1 percent by weight of mesityl oxide and is recycled to the work-up of acetone in the Hock process.

## Patentansprüche

1. Kontinuierliches Verfahren zur Rückgewinnung von Aceton aus einem Abfallstrom aus einer Acetonreinigungsstufe, wobei dieser Abfallstrom Mesityloxid und Aceton enthält, durch
i) Auftrennen des Abfallstroms in einer Trennvorrichtung zumindest in einen Strom, der Mesityloxid enthält, und einen weiteren Strom, der Aceton enthält,
ii) Aufkonzentrieren von Mesityloxid in dem mesityloxidhaltigen Strom und
iii) Zurückführen des aufkonzentrierten mesityloxidhaltigen Stroms zu der Trennvorrichtung und In-Kontakt-Bringen mit einem basischen oder sauren wässrigen Medium oder mit einem sauren Katalysator in Gegenwart von Wasser, wobei Mesityloxid zumindest teilweise zu Aceton hydrolysiert wird.

2. Verfahren nach Anspruch 1, wobei Aufkonzentrieren von Mesityloxid mittels Destillation erreicht wir.

3. Verfahren nach einem der Ansprüche 1 und 2, wobei der aufkonzentrierte mesityloxidhaltige Strom wenigstens 20, vorzugsweise 50 bis 85 Gew.-%, Mesityloxid enthält.

4. Verfahren nach einem der vorstehenden Ansprüche, wobei die Trennvorrichtung eine Destillationskolonne ist.

5. Verfahren nach einem der vorstehenden Ansprüche, wobei das basische oder saure wässrige Medium separat der Trennvorrichtung zugeführt wird.

6. Verfahren nach Anspruch 5, wobei der konzentrierte mesityloxidhaltige Strom mit Wasser gemischt und in der Trennvorrichtung mit einem sauren Katalysator, vorzugsweise einem immobilisierten heterogenen Katalysator, in Kontakt gebracht wird.

7. Verfahren nach einem der Ansprüche 1 bis 4, wobei das basische oder saure wässrige Medium bereits in dem Abfallstrom vorhanden ist.

8. Verfahren nach Anspruch 7, wobei die Konzentration des basischen oder sauren Mediums in dem Abfallstrom ≥ 0,1 Vol.-%, bezogen auf das Gesamtvolumen des Abfallstroms, ist.

9. Verfahren nach einem der Ansprüche 7 und 8, wobei der Abfallstrom zusätzlich eine oder mehrere organische Komponenten mit einem Siedepunkt, der höher als der von Aceton ist, enthält, das basische oder saure wässrige Medium ein basisches Medium ist und die Acetonrückgewinnung erreicht wird durch
a) kontinuierliches Zuführen dieses Abfallstroms zu einem mittleren Bereich der ersten Destillationskolonne,
b) Auftrennen des Abfallstroms in der ersten Destillationskolonne in
b1) wahlweise einen Kopfstrom, der Aceton enthält,
b2) einen Seitenstrom, der Mesityloxid, die organischen Komponenten und wahlweise restliches Aceton enthält, und
b3) einen Sumpfstrom, der das basische wässrige Medium enthält,
c) kontinuierliches Zuführen des Seitenstroms b2) zu einem mittleren Bereich der zweiten Destillationskolonne,
d) Auftrennen des Seitenstroms b2) in der zweiten Destillationskolonne in
d1) einen Kopfstrom, der Aceton enthält,
d2) einen Seitenstrom, der Mesityloxid enthält, und
d3) einen Sumpfstrom, der die organischen Komponenten mit einem Siedepunkt, der höher als der von Mesityloxid ist, enthält, und
e) kontinuierliches Rückführen des Seitenstroms d2) zu der ersten Destillationskolonne an einem Eintrittspunkt, der unterhalb des Abnahmepunkts für den Seitenstrom b2) ist.

10. Verfahren nach Anspruch 9, wobei der Seitenstrom b2) kontinuierlich einem Dekanter zugeführt wird, wobei der Seitenstrom in eine organische Phase, die kontinuierlich zu einem mittleren Bereich der zweiten Destillationskolonne geführt wird, und eine wässrige Phase, die zumindest teilweise zu der ersten Destillationskolonne zurückgeführt wird, aufgetrennt wird.

11. Verfahren nach einem der Ansprüche 9 und 10, wobei die erste Destillationskolonne Reaktionsböden enthält und der zurückgeführte mesityloxidhaltige Strom auf einen oder mehrere der Reaktionsböden geleitet wird.

12. Verfahren nach einem der Ansprüche 9 bis 11, wobei die Temperatur am Eintrittspunkt 30 bis 160°C, vorzugsweise 70 bis 110°C, beträgt.

13. Verfahren nach einem der Ansprüche 9 bis 12, wobei in der zweiten Destillationskolonne ein zweiter Seitenstrom d4), der organische Komponenten mit einem Siedepunkt, der niedriger ist als der von Mesityloxid, enthält, an einer Position oberhalb relativ zu der Position der Abnahme des Seitenstroms d2) entfernt wird.

14. Verfahren nach Anspruch 13, wobei der Seitenstrom d4) Benzol enthält.

15. Verfahren nach einem der vorstehenden Ansprüche, wobei der Abfallstrom aus der Acetonreinigung von Rohaceton, das nach dem Hock-Prozess erhalten wird, resultiert, wobei Rohaceton in einer Destillationskolonne abgetrennt wird, in der reines Aceton als Kopfstrom entfernt wird und der Abfallstrom als Sumpfstrom.

16. Verfahren nach Anspruch 15, wobei jegliche zurückgewonnenen Acetonströme miteinander vereint werden und in die Acetonreinigungskolonne zurückgeführt werden.

17. Verfahren zur Herstellung von Phenol und Aceton aus Cumol gemäß dem Hock-Prozess durch
A) Oxidieren von Cumol, um Cumolhydroperoxid zu bilden,
B) Unterwerfen von Cumolhydroperoxid einer säurenkatalysierten Spaltung, um eine Spaltungsphase zu erhalten, die Phenol und Aceton enthält,
C) Auftrennen von Phenol und Aceton,
D) Reinigen des Rohacetons aus Schritt C), resultierend in einen reinen Acetonproduktstrom und einen Abfallstrom, und
E) Rückgewinnung von Aceton aus dem Abfallstrom gemäß einem Verfahren nach einem der Ansprüche 1 bis 16.

18. Verfahren nach Anspruch 17, wobei in dem Rückgewinnungsschritt E) ein Verfahren nach einem der Ansprüche 9 bis 14 verwendet wird und wobei Sumpfstrom d3) Cumol enthält und in den Hock-Prozess zurückgeführt wird und
Sumpfstrom b3) das basische wässrige Medium und weniger als 2 Gew.-%, vorzugsweise weniger als 1 Gew.-%, Mesityloxid enthält und in die Aufarbeitung von Aceton in den Hock-Prozess zurückgeführt wird.

## Revendications

1. Procédé continu de récupération d'acétone à partir d'effluents provenant d'une étape de purification de l'acétone, lesdits effluents comprenant de l'oxyde de mésityle et de l'acétone, par
i) séparation des effluents dans un dispositif de séparation en au moins un courant comprenant de l'oxyde de mésityle et un autre courant comprenant de l'acétone :
ii) concentration de l'oxyde de mésityle dans le courant contenant l'oxyde de mésityle ; et
iii) recirculation du courant contenant l'oxyde de mésityle concentré vers le dispositif de séparation et mise en contact de celui-ci avec un milieu aqueux acide ou basique ou avec un catalyseur acide en présence d'eau, par laquelle l'oxyde de mésityle est au moins partiellement hydrolysé pour donner de l'acétone.

2. Procédé selon la revendication 1, dans lequel la concentration de l'oxyde de mésityle est obtenue par distillation.

3. Procédé selon l'une quelconque des revendications 1 et 2, dans lequel le courant contenant l'oxyde de mésityle concentré contient au moins 20 pourcents en poids d'oxyde de mésityle, de préférence 50 à 85 pourcents en poids.

4. Procédé selon l'une quelconque des revendications précédentes, dans lequel le dispositif de séparation est une colonne de distillation.

5. Procédé selon l'une quelconque des revendications précédentes, dans lequel le milieu aqueux acide ou basique est fourni séparément au dispositif de séparation.

6. Procédé selon la revendication 5, dans lequel le courant contenant l'oxyde de mésityle concentré est mélangé avec de l'eau et est mis en contact, dans le dispositif de séparation, avec un catalyseur acide, de préférence un catalyseur hétérogène immobilisé.

7. Procédé selon l'une quelconque des revendications 1 à 4, dans lequel le milieu aqueux basique ou acide est déjà présent dans les effluents.

8. Procédé selon la revendication 7, dans lequel la concentration du milieu basique ou acide dans les effluents est ≥0,1 pourcent en volume par rapport au volume total des effluents.

9. Procédé selon l'une quelconque des revendications 7 et 8, dans lequel les effluents comprennent, en outre, un ou plusieurs composants organiques dotés d'un point d'ébullition supérieur à celui de l'acétone, le milieu aqueux basique ou acide étant un milieu basique et la récupération de l'acétone étant obtenue par
a) la fourniture continue desdits effluents à la section médiane de la première colonne de distillation ;
b) la séparation des effluents dans la première colonne de distillation en
b1) un courant supérieur, de manière facultative, comprenant l'acétone ;
b2) un courant latéral comprenant l'oxyde de mésityle, les composants organiques et, de manière facultative, l'acétone résiduelle ; et
b3) un courant inférieur comprenant le milieu aqueux basique ;
c) la fourniture continue du courant latéral b2) à la section médiane de la seconde colonne de distillation ;
d) la séparation du courant latéral b2) dans la seconde colonne de distillation en
d1) un courant supérieur comprenant l'acétone ;
d2) un courant latéral comprenant l'oxyde de mésityle ; et d3) un courant inférieur comprenant les composants organiques dotés d'un point d'ébullition supérieur à celui de l'oxyde de mésityle ; et
e) la recirculation continue du courant latéral d2) vers la première colonne de distillation jusqu'à un point d'entrée en dessous du point de prélèvement pour le courant latéral b2).

10. Procédé selon la revendication 9, dans lequel le courant latéral b2) est fourni en continu à un décanteur, dans lequel il est séparé en une phase organique qui est fournie en continu à la section médiane de la seconde colonne de distillation, et une phase aqueuse qui est au moins partiellement recirculée à la première colonne de distillation.

11. Procédé selon l'une quelconque des revendications 9 et 10, dans lequel la première colonne de distillation comprend des plateaux de réaction et le courant contenant l'oxyde de mésityle recirculé est dirigé sur un ou plusieurs plateaux de réaction.

12. Procédé selon l'une quelconque des revendications 9 à 11, dans lequel la température au niveau du point d'entrée est comprise entre 30 et 160°C, de préférence entre 70 et 110°C.

13. Procédé selon l'une quelconque des revendications 9 à 12, dans lequel, dans la seconde colonne de distillation, un second courant latéral d4) comprenant des composants organiques dotés d'un point d'ébullition inférieur à celui de l'oxyde de mésityle est prélevé au niveau d'une position vers le haut par rapport à la position de prélèvement du courant latéral d2).

14. Procédé selon la revendication 13, dans lequel le courant latéral d4) comprend du benzène.

15. Procédé selon l'une quelconque des revendications précédentes, dans lequel les effluents proviennent de la purification de l'acétone brute obtenue par le procédé de Hock, par lequel l'acétone brute est séparée dans une colonne de distillation, dans laquelle l'acétone pure est prélevée comme courant supérieur et les effluents comme courant inférieur.

16. Procédé selon la revendication 15, dans lequel tout courant d'acétone récupéré est combiné et recirculé dans la colonne de purification de l'acétone.

17. Procédé de production de phénol et d'acétone à partir de cumène selon le procédé de Hock par :
A) oxydation du cumène pour former de l'hydroperoxyde de cumène ;
B) soumission de l'hydroperoxyde de cumène à un clivage catalysé par acide produisant une phase de clivage comprenant du phénol et de l'acétone ;
C) séparation du phénol et de l'acétone ;
D) purification de l'acétone brute provenant de l'étape C) produisant un courant de produit d'acétone pure et des effluents ; et
E) récupération de l'acétone provenant des effluents suivant un procédé selon l'une quelconque des revendications 1 à 16.

18. Procédé selon la revendication 17, dans lequel, lors de l'étape de récupération E), on utilise un procédé selon l'une quelconque des revendications 9 à 14 et dans lequel :
le courant inférieur d3) comprend du cumène et est recirculé selon le procédé de Hock
et
le courant inférieur b3) comprend le milieu aqueux basique et moins de 2 pourcents en poids, de préférence moins de 1 pourcent en poids, d'oxyde de mésityle et est recirculé vers le traitement conclusif de l'acétone selon le procédé de Hock.
